# EUROPEAN PATENT SPECIFICATION

(11) **EP 4 521 115 B1**
(45) Date of publication and mention of the grant of the patent: **14.10.2026**
(21) Application number: 24166517.3
(22) Date of filing: 10.06.2021
(51) Int. Cl.: C12Q 1/6837

(54) **METHODS OF DETECTING METHYLATED CPG**
VERFAHREN ZUM NACHWEIS VON METHYLIERTEM CPG
PROCÉDÉS DE DÉTECTION DE CPG MÉTHYLÉ

(30) Priority: 10.06.2020 US 202063037020 P
(43) Date of publication of application: 12.03.2025
(62) Document number(s) of the earlier application(s) in accordance with Art. 76 EPC: 21821437.7 / 4 165 213
(73) Proprietor: Ramot at Tel-Aviv University Ltd., Tel-Aviv 6139201 (IL)
(72) Inventor: EBENSTEIN, Yuval, 6139201 Tel Aviv (IL); ZIRKIN, Shahar, 6139201 Tel Aviv (IL); MICHAELI-HOCH, Yael, 6139201 Tel Aviv (IL); GILAT, Noa, 6139201 Tel Aviv (IL); FELDSTEIN, Orit, 6139201 Tel Aviv (IL); BEN-MOSHE, Adaya, 6139201 Tel Aviv (IL)
(74) Representative: Witek, Rafal

(56) References cited:
- WO-A1-2018/029693
- US-A1- 2006 292 585
- GITAN R S ET AL: "Methylation-Specific Oligonucleotide Microarray: A New Potential for High-Throughput Methylation Analysis", GENOME RESEARCH, COLD SPRING HARBOR LABORATORY PRESS, US, vol. 12, no. 1, 1 January 2002 (2002-01-01), pages 158 - 164, XP003015597, ISSN: 1088-9051, DOI: 10.1101/GR.202801
- N. KIMURA: "Methylation profiles of genes utilizing newly developed CpG island methylation microarray on colorectal cancer patients", NUCLEIC ACIDS RESEARCH, vol. 33, no. 5, 8 March 2005 (2005-03-08), pages e46 - e46, XP055108550, DOI: 10.1093/nar/gni046

## Description

### FIELD AND BACKGROUND OF THE INVENTION

The present invention, in some embodiments thereof, relates to methods of detecting methylated CpG.

Despite having an identical nucleotide sequence, the DNA of each cell type in the body carries unique epigenetic signature correlating with its gene expression profile. In particular, DNA methylation, serving to repress gene expression, is a fundamental aspect of tissue identity and state. Methylation has been reported to exhibit tissue-specific patterns, to correlate with gene regulation and expression, and to be suitable as a biomarker for multiple types of cancer and other pathologies. Furthermore, as blood levels of cell-free circulating DNA (cfDNA) derived from apoptotic and necrotic cells are known to increase under a variety of pathological conditions including cancer, autoimmune diseases, stroke and various organ injuries; the methylation pattern of cfDNA may be used to determine its tissue of origin and hence to infer cell death in the source organ.

Up to date, several methods have been developed for the quantification of DNA methylation, such as whole genome bisulfite sequencing, methylation specific PCR and Illumina microarray slide for specific CpG. However, these methods are either laborious, complex or expensive to perform, or are inaccurate and insensitive enough to meet the requirements for clinical use.

US2006/292585A1 discloses a method of determining CpG methylation status in a DNA sample comprising ligating DNA sample fragments to one or more adaptor sequences before treatment with bisulfite. The bisulfite treated sample may then be amplified by PCR using primers that are complementary to the adaptors. The amplified fragments are hybbridized onto a probe array. The amplified fragments are labelled before hybridization.

WO2018/029693A1 discloses CpG DNA methylation detection at the single-molecule level by DNA-methyltransferase assisted labelling.

Additional background art includes International Applications Publication Nos. WO2019/234753, WO2018/029693, WO2017/081689 and WO2014/191981.

### SUMMARY OF THE INVENTION

According to the present invention there is provided a method of determining CpG methylation status in a DNA sample, the method comprising:
(a) subjecting the DNA sample to bisulfite conversion;
(b) amplifying the DNA sample following the (a) by a PCR using adaptors ligation, to obtain an amplified DNA sample;
(c) labeling CpG sites in said amplified DNA sample with a label using a CpG methylation-sensitive methyltransferase (MTase), to obtain a labeled DNA sample;
(d) contacting the amplified DNA sample on an array comprising a plurality of probes for the DNA under conditions which allow specific hybridization between the plurality of probes and the DNA; and
(e) detecting hybridization, wherein an amount of the label is indicative of the CpG methylation status in the DNA sample.

According to some embodiments of the invention, the DNA sample comprises DNA fragments.

According to some embodiments of the invention, the method comprising fragmenting the DNA so as to obtain DNA fragments prior to the (d).

According to some embodiments of the invention, the DNA fragments are about 100-300 nucleotides long.

According to some embodiments of the invention, a concentration of the DNA prior to amplification in the sample is ≥ 0.01 pg per ml.

According to some embodiments of the invention, the DNA is cellular DNA.

According to some embodiments of the invention, the method comprising lysing the cells of the cellular DNA prior to the (a).

According to some embodiments of the invention, the DNA is cell-free DNA (cfDNA).

According to some embodiments of the invention, the amplifying comprises whole DNA amplification.

According to some embodiments of the invention, the method further comprising ligating the adaptors to the DNA sample prior to the subjecting.

According to some embodiments of the invention, the adaptors comprise a methylated cytosine nucleotide.

According to some embodiments of the invention, the adaptor are devoid of a methylated CpG site.

According to some embodiments of the invention, the adaptors are devoid of an unmethylated cytosine nucleotide.

According to some embodiments of the invention, one of the adaptors comprises SEQ ID NO: 1.

According to some embodiments of the invention, one of the adaptors comprises SEQ ID NO: 3.

According to some embodiments of the invention, the PCR is effected using random primers.

According to some embodiments of the invention, the amplifying is effected by Multiple Displacement Amplification.

According to some embodiments of the invention, the method further comprising labeling with the CpG site indicative label.

According to some embodiments of the invention, the labeling comprises fluorescently labeling.

According to some embodiments of the invention, the labeling comprises enzymatically labeling.

According to some embodiments of the invention, the labeling is effected by a methyltransferase (MTase) or a T4-Beta-glucosyl transferase (T4-βGT).

According to some embodiments of the invention, the MTase is selected from the group consisting of M.TaqI, M.HhaI, M.HpaII, M.MspI, M.SssI and M.MpeI or a mutant or derivative thereof.

According to an aspect of the present invention there is provided a method of identifying DNA having a methylation pattern distinctive of a cell or tissue type or state, the method comprising determining CpG methylation status in a DNA sample according to the method, wherein the CpG methylation status is indicative of the cell or tissue type or state.

According to some embodiments of the invention, the cell comprises a pathologic cell.

According to some embodiments of the invention, the pathologic cell is a cancerous cell, a cell associated with a neurological disease, a cell associated with an autoimmune disease or a grafted cell.

According to some embodiments of the invention, the pathologic cell is a cancerous cell.

According to some embodiments of the invention, the cell has been exposed to an agent selected from the group consisting of chemotherapy, chemical treatment, radiation and DNA damaging agent.

According to an aspect of the present invention there is provided a method of diagnosing a pathology in a subject, the method comprising obtaining a biological sample of the subject and identifying DNA having a methylation pattern distinctive of a cell or tissue type or state according to the method, wherein presence and/or level above a predetermined threshold of the DNA having the methylation pattern distinctive of the cell or tissue type or state is indicative of a pathology associated with the cell or tissue in the subject.

According to an aspect of some embodiments of the present invention there is provided a method of treating a pathology in a subject in need thereof, the method comprising:
(i) diagnosing the pathology in the subject according to the method; and wherein the pathology is indicated
(ii) treating the pathology in the subject.

According to an aspect of the present invention there is provided a method of monitoring a treatment for a pathology in a subject in need thereof, the method comprising obtaining a biological sample of the subject and identifying DNA having a methylation pattern distinctive of a cell or tissue associated with the pathology according to the method, wherein a decrease above a predetermined threshold of the DNA having the methylation pattern distinctive of the cell or tissue following treatment as compared to same prior to treatment indicates efficacy of treatment of the pathology in the subject.

According to some embodiments of the invention, the sample is a body fluid sample.

According to some embodiments of the invention, the fluid is selected from the group consisting of blood, plasma, serum, saliva, tears and urine.

According to an aspect of the present invention there is provided a method of detecting death of a cell or tissue of interest in a subject comprising determining whether cell-free DNA (cfDNA) comprised in a fluid sample of the subject is derived from the cell or tissue of interest, wherein the determining is effected by the method, wherein presence and/or level above a predetermined threshold of the DNA having a methylation pattern distinctive of the cell or tissue of interest is indicative of death of the cell or tissue of interest.

According to some embodiments of the invention, when death of the cell or tissue is associated with a pathology, the method further comprises diagnosing the pathology.

According to some embodiments of the invention, the pathology is cancer, neurological disease, autoimmune disease or graft injury.

According to some embodiments of the invention, the pathology is cancer.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

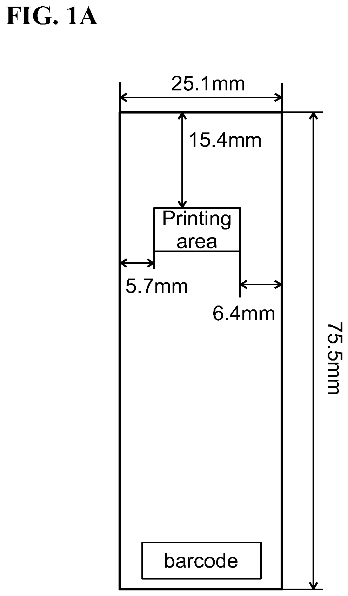

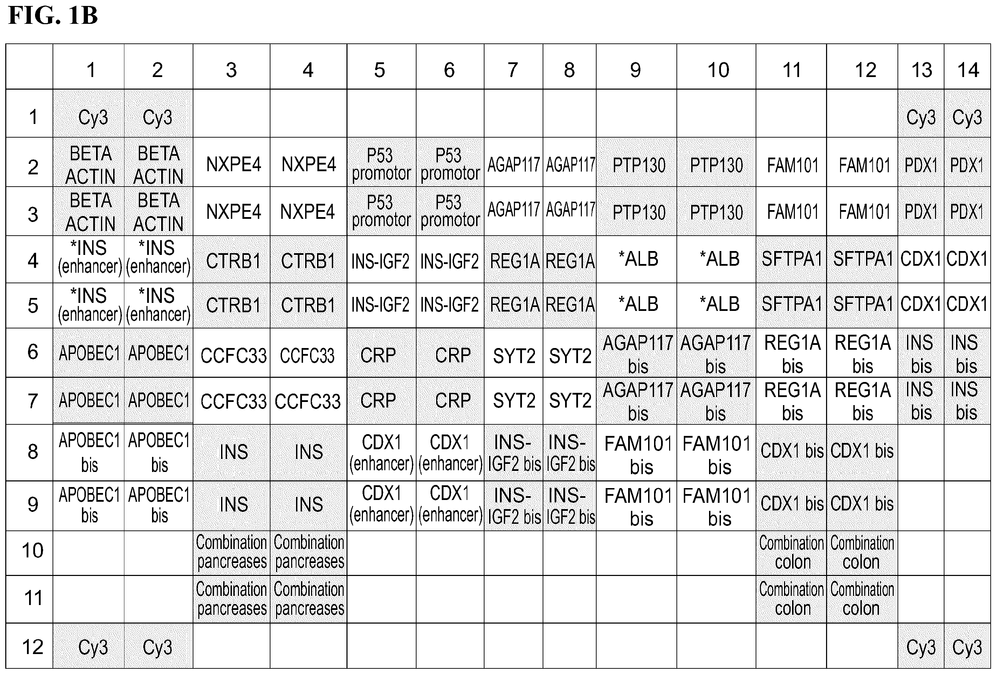

FIGs. 1A-B demonstrate an example of a custom-made array that may be used with specific embodiments of the invention. Figure 1A shows an entire microarray slide, containing the barcode and printed area. Figure 1B shows an enlargement of the printed area, which contains quadruplicates of each of the designed spots (pixels). Each pixel contains multiple short DNA sequences, designed to capture specific DNA fragments.

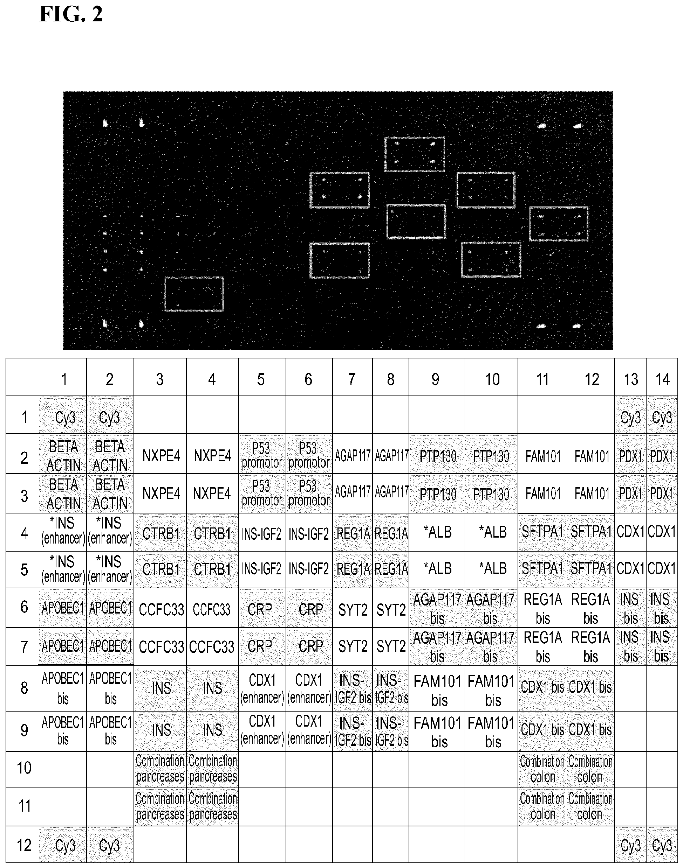

FIG. 2 shows an image of a scan of the printed area of a microarray slide. cfDNA was treated according to the procedure describe in the Examples section which follows. The labeled DNA was hybridized to the custom-made microarray slide. The light up spots are marked with gray rectangles, representing the areas where labeled DNA was captured. A map of the marked areas appears below the scan image, and the respective areas are marked with gray rectangles.

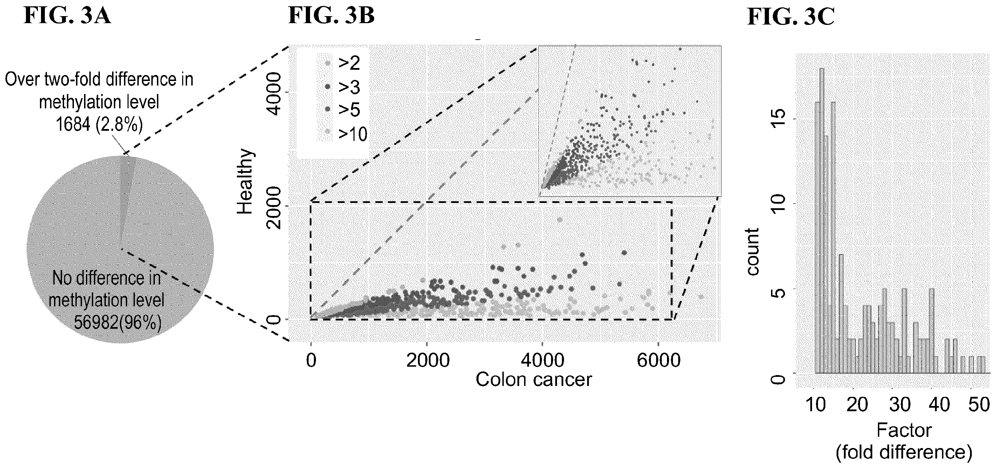

FIGs. 3A-C demonstrate identification of potential biomarkers in colon cancer samples. cfDNA was treated according to the procedure describe in the Examples section which follows. The labeled DNA was hybridized to the Human Genome CGH Microarray (Agilent) microarray slide. Following, data from different arrays was normalized and loci with over two-fold differential methylation between healthy and colon cancer samples were indicated as potential biomarkers. Figure 3A shows identification of 1684 such loci (out of ~60,000 loci). Figure 3B shows the change-fold distribution of the 1684 potential bio-markers. Figure 3C shows a histogram of the distribution of biomarkers with biomarkers higher than 10-fold change in methylation level.

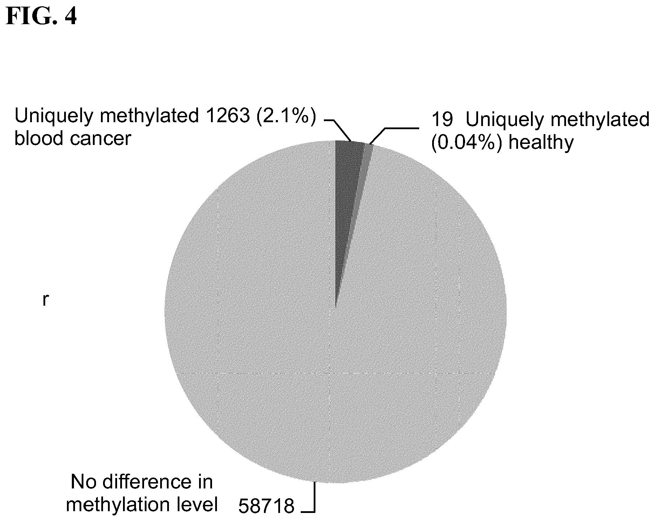

FIG. 4 demonstrates identification of potential biomarkers in blood cancer samples. cfDNA was treated according to the procedure describe in the Examples section which follows. The labeled DNA was hybridized to the Human Genome CGH Microarray (Agilent) microarray slide. Following, data from different arrays was normalized and loci with over two-fold differential methylation between healthy and blood cancer samples were indicated as potential biomarkers. 1263 such loci (out of ~60,000 loci) were identified.

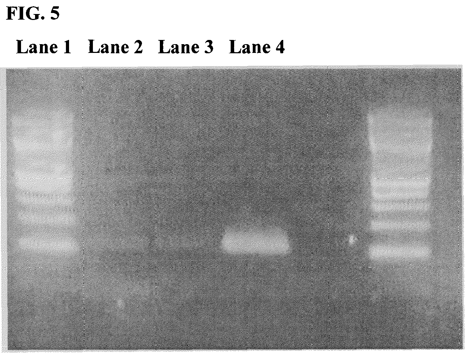

FIG. 5 is a gel electrophoresis image demonstrating the comparison between adaptors' ligation based amplification (protocol 1) and isothermal nucleic acid amplification (protocol 2) following sodium bisulfite treatment. Lane 1 - 100 bp ladder; Lanes 2-3 - a specific DNA segment amplified from the DNA templates obtained by amplification protocol 2 (i.e. Illumina based) of DNA samples from blood (lane 2) or U2OS cell line (lane 3); Lane 4 - a specific DNA segment amplified from the DNA templates obtained by amplification protocol 1 of cfDNA.

### DESCRIPTION OF SPECIFIC EMBODIMENTS OF THE INVENTION

The present invention, in some embodiments thereof, relates to methods of detecting methylated CpG.

Methylation has been reported to exhibit tissue-specific patterns, to correlate with gene regulation and expression, and to be suitable as a biomarker for multiple types of cancer and other pathologies. In an effort to overcome limitations of current methods for quantification of DNA methylation, such as inaccuracy and lack of sensitivity, the present inventors have developed a novel method for determining methylation status of DNA. This method takes advantage of bisulfite conversion which results in conversion of all the unmethylated cytosine nucleotides in a DNA sample into uracil nucleotides, followed by an amplification process which results in conversion of all the uracil nucleotides into thymidine nucleotides allowing further labeling and detection of only CpG sites that were methylated in the original DNA sample.

Thus, according to an aspect of the invention, there is provided a method of determining CpG methylation status in a DNA sample, the method comprising:
(a) subjecting the DNA sample to bisulfite conversion;
(b) amplifying said DNA sample following said (a) to obtain an amplified DNA sample;
(c) labeling CpG sites in said amplified DNA sample with a label to obtain a labeled DNA sample;
(d) contacting said labeled DNA sample on an array comprising a plurality of probes for said DNA under conditions which allow specific hybridization between said plurality of probes and said DNA; and
(e) detecting said hybridization, wherein an amount of said label is indicative of the CpG methylation status in said DNA sample.

According to an additional or an alternative aspect of the invention, there is provided a method of determining CpG methylation status in a DNA sample, the method comprising:
(a) subjecting the DNA sample to bisulfite conversion;
(b) amplifying said DNA sample following said (a) by a PCR using adaptors ligation, to obtain an amplified DNA sample;
(c) contacting said amplified DNA sample on an array comprising a plurality of probes for said DNA under conditions which allow specific hybridization between said plurality of probes and said DNA;
(d) detecting hybridization based on a CpG site indicative label, wherein an amount of said label is indicative of the CpG methylation status in said DNA sample.

As used herein the term "CpG site" refers to a region of DNA where a cytosine nucleotide occurs next to a guanine nucleotide in the linear sequence of bases along its length, the cytosine (C) being separated by only one phosphate (p) from the guanine (G). DNA regions that have a higher concentration of CpG sites are known as "CpG islands".

Herein, the phrase "CpG methylation status" refers to the pattern of methylation of cytosine nucleotides in the context of CpG sites (or islands) in a DNA sample, and thus refers to the presence or absence of methylated cytosine nucleotides.

Examples of methylation of CpG that may be detected according to embodiments of the invention include, without limitation, unmethylated CpG, 5-methylcytosine, 5-hydroxymethylcytosine, 5-carboxycytosine and/or 5-formylcytosine.

The DNA can be a mammalian DNA (e.g., human) or plant DNA in which CpG modifications typically occur or a synthetic DNA in which CpG modifications may be artificially added.

According to an embodiment of the invention, the DNA molecule is a complementary polynucleotide sequence (cDNA) to which CpG modifications have been artificially added, a genomic polynucleotide sequence and/or a composite polynucleotide sequences (e.g., a combination of the above).

According to specific embodiments, the DNA sample is obtained from a biological sample of a subject. Such a biological sample may comprise a tissue sample or a body fluid sample including, but not limited to, tissue biopsy, tissue section, formalin fixed paraffin embedded (FFPE) specimens, blood, plasma, serum, bone marrow, cerebro-spinal fluid, tears, sweat, lymph fluid, saliva, nasal swab or nasal aspirate, sputum, bronchoalveolar lavage, breast aspirate, pleural effusion, peritoneal fluid, glandular fluid, amniotic fluid, cervical swab or vaginal fluid, ejaculate, semen, prostate fluid, urine, pus, conjunctival fluid, duodenal juice, pancreatic juice, bile, and stool.

According to specific embodiments, the DNA sample is obtained from a body fluid sample. In specific embodiments, fluid is selected from the group consisting of blood, plasma, serum, sperm, milk, urine, saliva and cerebral spinal fluid. In particular embodiments the fluid is selected from the group consisting of blood, plasma, urine, tears and serum. According to specific embodiments, the fluid is selected from the group consisting of blood, plasma, serum, saliva, tears and urine. According to specific embodiments, the fluid is selected from the group consisting of blood, plasma, serum, saliva and urine.

It will be appreciated that the methods disclosed herein are suitable for highly sensitive detection of DNA methylation pattern in any properly prepared sample, and not exclusively in biological samples, or of biological material. Thus, in some embodiments, the sample is an aqueous sample of a nucleic acid.

According to specific embodiments, the method comprises extracting the DNA e.g. from the biological sample.

Methods of DNA extraction are well known in the art and are further described in the Examples section which follows. DNA extraction kits are also commercially available, for example the QiaAmp tissue kits. Some body fluids should be pre-treated under appropriate condition prior to DNA extraction. For example, if a blood sample is used, anti-coagulants contained in whole blood should be able to inhibit DNAse activity. A suitable anti-coagulant may be a chelating agent such as EDTA that prevents both DNAse-caused DNA degradation and clotting of the whole blood samples. If other body fluid samples such as sputum are used, cells in these kinds of samples can be collected by the procedures described in prior art. For example, collection of cells in a urine sample can simply be achieved by simply centrifugation, while collection of cells in a sputum sample requires DTT treatment of sputum followed by filtering through a nylon gauze mesh filter and then centrifugation. If a stool sample is used, a stool stabilizing and homogenizing reagents should be added to stabilize DNA and remove stool particles. Human DNA fraction from total stool DNA then can be primarily isolated or purified using commercially available stool DNA isolation kits such as Qiagen DNA Stool Mini Kit (using the protocol for human DNA extraction) or be captured by methyl-binding domain (MBD)-based methylated DNA capture methods after total DNA isolation [Zhou H et al., Clinical Chemistry, 2007].

In some embodiments, the sample comprises cells and/or tissues, and DNA of the sample is cellular DNA (e.g. genomic DNA). Cellular DNA can be obtained after its release from the cell. Hence, according to specific embodiments, the method comprising lysing the cells comprised in the sample. In some embodiments, cells are disrupted mechanically (e.g. sonication, pressure, impact-e.g. glass beads, etc), chemically (detergents such as SDS, Triton, etc) or thermally (heating). In some embodiments, the cellular contents are then subjected to denaturation of nucleoproteins and/or inactivation of cellular enzymes, for example, by guanidinium thiocyanate, phenol extraction, proteinase, chelation and/or detergent treatment. Following denaturation/inactivation, in some embodiments, the cell lysate is further cleansed of contaminants, for example, by salting out, organic extraction, PEG extraction, chelation and/or adsorption (e.g. diatomaceous earth).

Finally, DNA may be precipitated from the cell lysate for purification. Methods for precipitation of DNA include, but are not limited to alcohol (e.g. ethanol, isopropanol) precipitation, sodium acetate + alcohol, and magnetic beads (DNA can be adsorbed onto silica-coated surfaces). DNA can then be processed for detection of profiles of epigenetic modifications according to the methods of the invention.

In particular embodiments, the sample comprises cell-free DNA (cfDNA).

In specific embodiments, the sample is a serum or plasma sample comprising cfDNA, and the DNA of the sample is cfDNA.

It will be appreciated that, in some embodiments, in cases the target DNA for analysis is cell-free DNA (cfDNA), either tissue or cellular components are removed from the samples, leaving cfDNA, or the samples are processed for characterization of the profile of CpG methylation without removal of cells or cellular debris, for example, when the sample is of a bodily fluid.

In some embodiments, the DNA of the sample is in DNA fragments.

The DNA fragments can be in the range of 20-2000 nucleotides in length. In some embodiments, the DNA fragments of the sample are 50-1500 nucleotides long, 100-1200 nucleotides long, 150- 1000 nucleotides long, 1000-1500 nucleotides long, 50-300 nucleotides long, about 100, about 200, about 300, about 400, about 500, about 600, about 700, about 800, about 900, about 100, about 1100, about 1200, about 1300, about 1400, about 1500, about 1600, about 1700, about 1800, about 1900 or about 2000 nucleotides long. In specific embodiments, the DNA fragments are 1000-1500 nucleotides long, 100-300 nucleotides long or about 200 nucleotides long.

In some embodiments, the DNA of the sample is fragmented prior to contacting the sample on the array. Fragmenting the DNA of a sample can be effected by methods known in the art, including but not exclusively enzymatic (e.g. endonuclease) fragmentation, acoustic fragmentation, sonication, centrifugal shearing, point-sink shearing, needle (hypodermic) shearing and the like. In specific embodiments, the DNA is fragmented by shearing. Some methods of DNA fragmentation are detailed in PCT Publication WO 2016/178207.

In some embodiments, the CpG methylation modifications of interest in the DNA of the samples are present on a plurality of different fragments of the sample DNA.

According to specific embodiments, the concentration of the DNA in the sample prior to amplification is at least 0.0001 pg / ml, at least 0.001 pg / ml, at least 0.01 pg / ml, at least 0.1 pg / ml, at least 1 pg / ml, at least 10 pg / ml, at least 0.1 ng / ml, at least 1 ng / ml, at least 10 ng / ml or at least 100 ng / ml.

According to a specific embodiment, the concentration of the DNA in the sample prior to amplification is at least 0.01 pg / ml.

According to a specific embodiment, the concentration of the DNA in the sample prior to amplification is 0.1-10 ng/µL.

As used herein, the term "bisulfite conversion" refers to a process of contacting DNA with bisulfite under conditions that allow deamination of unmethylated cytosine nucleotides to uracil nucleotides, while preserving the methylated cytosine nucleotides unchanged. Examples of reagents for bisulfite conversion include sodium bisulfite, magnesium bisulfite, and trialkylammonium bisulfite.

The bisulfite conversion conditions which include, but not limited to, reagents, temperature, buffer, salt, ionic strength, pH, and the like may readily be selected and/or designed by one skilled in the art. Bisulfite conversion kits are also commercially available from e.g. ZYMO.

As used herein, the term "amplifying" or "amplification" refers to a process that increases DNA sequences in a sample by producing multiple (i.e., at least 2) copies of the sequences.

According to specific embodiments, the amplification process results in amplification of the total DNA in the sample in a non-sequence specific manner (e.g. using adaptors or random primers). That is, the amplification process results in the uniform amplification of the entire DNA molecules or fragments in the sample (i.e. whole DNA amplification).

According to other specific embodiments, the amplification process results in the representation of a population of specific DNA sequences in the sample by producing multiple copies of the desired sequences.

Methods for DNA amplification which can be used with specific embodiments of the invention are known in the art and include, but are not limited to, polymerase chain reaction (PCR), ligase chain reaction (LCR), isothermal nucleic acid amplification and Multiple Displacement Amplification (MDA). In a typical PCR amplification reaction, a DNA sequence of interest is often amplified at least fifty thousand fold in amount over its amount in the starting sample.

A typical amplification reaction is carried out by contacting a forward and reverse primer (a primer pair) to the sample DNA together with any additional amplification reaction reagents under conditions which allow amplification of the target sequence.

The amplification conditions which include, but not limited to, reagents, temperature, buffer, salt, ionic strength, pH, enzymes and the like may readily be selected and/or designed by one skilled in the art.

Thus, for example, amplification conditions generally comprise conditions that promote annealing and/or extension of primer sequences. Such conditions are well-known in the art and depend on the amplification method selected. Thus, for example, in a PCR reaction, amplification conditions generally comprise thermal cycling, i.e., cycling of the reaction mixture between two or more temperatures. In isothermal amplification reactions, amplification occurs without thermal cycling although an initial temperature increase may be required to initiate the reaction.

According to specific embodiments, the amplification is effected a PCR.

According to specific embodiments, the amplification is effected using adaptors ligated to the DNA prior to the amplification.

Thus, according to specific embodiments, the method comprises ligating the adaptors to the DNA sample prior to amplifying.

According to specific embodiments, the method comprises ligating the adaptors to the DNA sample prior to subjecting the sample to bisulfite conversion.

Such an adaptor is typically a short, chemically synthesized, single-stranded or double-stranded oligonucleotide that can be ligated to the ends of another DNA molecule. The art of adaptors ligation is well known to the skilled in the art. Ligation kits and reagents are also available commercially from e.g. New England Biolabs (NEB), Sigma-Aldrich, Thermo Fisher.

Following, amplification of the DNA is effected using primers complementary to the adaptors.

According to specific embodiments, the primers or adaptors used for amplification may contain methylated cytosine nucleotides, but not in the context of CpGs, so as to avoid labeling of the primers or adaptors.

According to specific embodiments, all cytosine nucleotides in the primers or adaptors are methylated.

According to specific embodiments, the primers or adaptors used for amplification do not comprise unmethylated cytosine nucleotides e.g. in the context of CpGs.

According to specific embodiments, the primers or adaptors are devoid of CpG sites.

According to specific embodiments, typically, two adaptors are used (one for the 5' end and on for the 3' end). The two adaptors' sequences in use may partially complement one another (between 5-10 bases) for a better ligation process.

Non-limiting examples of adaptor and their respective primers that can be used with specific embodiments include SEQ ID NO: 1-4 or SEQ ID NO: 5-8.

| | | |
|---|---|---|
| Adaptor 3 | 5-GTCTAGGGAACATAGGATCAGGACT | SEQ ID NO: 5 |
| Primer 3 | GTC TAG GGA ACA TAG GATGTC | SEQ ID NO: 6 |
| Adaptor 4 | 5- *P- GGA GAC TAT TGG TGA CTA CAA CTT G | SEQ ID NO: 7 |
| Primer 5 | CAAGTTGTAGTCACCAATAGTC | SEQ ID NO: 8 |

| | | |
|---|---|---|
| * P at the beginning of the sequence represents a phosphate group | | |

According to specific embodiments, one of the adaptors comprises SEQ ID NO: 1.

According to specific embodiments, one of the adaptors consists of SEQ ID NO: 1.

According to specific embodiments, one of the adaptors comprises SEQ ID NO: 3.

According to specific embodiments, one of the adaptors consists of SEQ ID NO: 3.

The term "label", "labeling" or "labeling agent" refers to a detectable moiety which can be attached to a DNA sequence and is indicative of a CpG site. Such labels are known to the skilled in the art and non-limiting examples are further described hereinbelow and in the Examples section that follows. Labels suitable with some embodiments of the invention are commercially available for example from Illumina (e.g. Infinium MethylationEPIC BeadChip Cat No. WG-317- 1002).

According to specific embodiments, the label is a detectable moiety which can be attached specifically to a CpG site in a DNA sequence. According to specific embodiments, the label recognizes a non-methylated C in the CpG site and does not recognize a methylated C in the CpG site.

Exemplary labels which are suitable for use with specific embodiments include, but are not limited to, a fluorescent agent, a radioactive agent, a magnetic agent, a chromophore, a bioluminescent agent, a chemiluminescent agent, a phosphorescent agent and a heavy metal cluster, a bulky adduct, an oligonucleotide as well as any other known detectable agents.

According to specific embodiments, the label is detectable by spectrophotometric measurements, and/or which can be utilized to produce optical imaging. Such labels include, for example, chromophores, fluorescent agents, phosphorescent agents, and heavy metal clusters.

As used herein, the term "chromophore" refers to a chemical moiety that, when attached to another molecule, renders the latter colored and thus visible when various spectrophotometric measurements are applied.

The phrase "fluorescent agent" refers to a compound that emits light at a specific wavelength during exposure to radiation from an external source.

The phrase "phosphorescent agent" refers to a compound emitting light without appreciable heat or external excitation as by slow oxidation of phosphorous.

A heavy metal cluster can be for example a cluster of gold atoms used, for example, for labeling in electron microscopy techniques (e.g., AFM).

The term "bioluminescent agent" describes a substance which emits light by a biochemical process.

The term "chemiluminescent agent" describes a substance which emits light as the result of a chemical reaction.

According to some embodiments of the invention, the label is a fluorescent labeling agent.

A fluorescent label can be a protein, quantum dots or small molecules. Common dye families include, but are not limited to Xanthene derivatives: fluorescein, rhodamine, Oregon green, eosin, Texas red etc.; Cyanine derivatives: cyanine, indocarbocyanine, oxacarbocyanine, thiacarbocyanine and merocyanine; Naphthalene derivatives (dansyl and prodan derivatives); Coumarin derivatives; oxadiazole derivatives: pyridyloxazole, nitrobenzoxadiazole and benzoxadiazole; Pyrene derivatives: cascade blue etc.; BODIPY (Invitrogen); Oxazine derivatives: Nile red, Nile blue, cresyl violet, oxazine 170 etc.; Acridine derivatives: proflavin, acridine orange, acridine yellow etc.; Arylmethine derivatives: auramine, crystal violet, malachite green; CF dye (Biotium); Alexa Fluor (Invitrogen); Atto and Tracy (Sigma Aldrich); FluoProbes (Interchim); Tetrapyrrole derivatives: porphin, phtalocyanine, bilirubin; cascade yellow; azure B; acridine orange; DAPI; Hoechst 33258; lucifer yellow; piroxicam; quinine and anthraqinone; squarylium; oligophenylenes; and the like.

Other fluorophores include: Hydroxycoumarin; Aminocoumarin; Methoxycoumarin; Cascade Blue; Pacific Blue; Pacific Orange; Lucifer yellow; NBD; R-Phycoerythrin (PE); PE-Cy5 conjugates; PE-Cy7 conjugates; Red 613; PerCP; TruRed; FluorX; Fluorescein; BODIPY-FL; TRITC; X-Rhodamine; Lissamine Rhodamine B; Texas Red; Aliaphycocyanin; APC-Cy7 conjugates.

Alexa Fluor dyes (Molecular Probes) include: Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, and Alexa Fluor 790.

Cy Dyes (GE Heathcare) include Cyt, Cy3, Cy3B, Cy3.5, Cy5, Cy5.5 and Cy7.

Nucleic acid probes include Hoechst 33342, DAPI, Hoechst 33258, SYTOX Blue, ChromomycinA3, Mithramycin, YOYO-1, Ethidium Bromide, Acridine Orange, SYTOX Green, TOTO-1, TO-PRO-1, TO-PRO: Cyanine Monomer, Thiazole Orange, Propidium Iodide (PI), LDS 751, 7-AAD, SYTOX Orange, TOT0-3, TO-PR0-3, and DRAQ5.

Cell function probes include Indo-1, Fluo-3, DCFH, DHR, SNARF.

Fluorescent proteins include Y66H, Y66F, EBFP, EBFP2, Azurite, GFPuv, T-Sapphire, Cerulean, mCFP, ECFP, CyPet, Y66W, mKeima-Red, TagCFP, AmCyan1, mTFP1, S65A, Midoriishi Cyan, Wild Type GFP, S65C, TurboGFP, TagGFP, S65L, Emerald, S65T (Invitrogen), EGFP (Ciontech), Azami Green (MBL), ZsGreen1 (Clontech), TagYFP (Evrogen), EYFP (Clontech), Topaz, Venus, mCitrine, YPet, Turbo YFP, ZsYellow1 (Clontech), Kusabira Orange (MBL), mOrange, mKO, TurboRFP (Evrogen), tdTomato, TagRFP (Evrogen), DsRed (Clontech), DsRed2 (Clontech), mStrawberry, TurboFP602 (Evrogen), AsRed2 (Clontech), mRFP1, J-Red, mCherry, HcRed1 (Clontech), Katusha, Kate (Evrogen), TurboFP635 (Evrogen), mPlum, and mRaspberry.

Exemplary fluorescent labels include, but are not limited to, Alexa fluor dyes, Cy dyes, Atto dyes, TAMRA dyes and the like.

Labeling a DNA molecule with the label may optionally be effected using suitable reagents, such as are known in the art. It is to be noted that, according to specific embodiments, the label is attached to the DNA molecule, for example by means of click chemistry and that the reagents used for the reaction are derivatives of the labeling agent, which include a reactive group.

According to specific embodiments, the label is attached to the DNA using an enzyme (enzymatic labeling).

In specific embodiments, the label is attached to the DNA molecule using a methyltransferase (MTase) or a Beta-glycosyle transferase (βGT) and a cofactor to functionalize the DNA, and a label is then covalently attached to the DNA via the functional group. In some embodiments, following functionalization, the label is attached using a click reaction, optionally a copper-free click reaction.

In specific embodiments, the label is attached to the DNA molecule using a methyltransferase (MTase).

As used herein, the term "methyltransferase enzyme, MTase" refers to an enzyme which transfers the activated methyl group from the natural cofactor S-adenosyl-L-methionine (AdoMet or SAM) to adenine-N6, cytosine-N4 or cytosine-C5 within specific double-stranded DNA sequences ranging from two to eight base pairs.

Preferably, the DNA methyltransferase is an enzyme capable of methylating DNA. More preferably, the methyltransferase is a DNA cytosine-C5 methyltransferase that uses a covalent activation mechanism for the transfer of the methyl group on the C5 position of a target cytosine residue.

According to specific embodiments, the methyltransferase is a CpG methylation-sensitive MTase, i.e. only recognizes the non-methylated C base in the context of a CpG site (i.e. a region of DNA where a cytosine nucleotide is followed by a guanine nucleotide in the linear sequence of bases along its 5' → 3' direction).

In one embodiment, the CpG-methylation sensitive MTase is selected from the group consisting of M.TaqI, M.HhaI, M.HpaII, M.MspI, M.SssI and M.MpeI or a functional mutant or derivative thereof.

Non-limiting examples of MTase mutants that can be used with specific embodiments of the invention include M.Mpel Q136A/N374A and M.SssIQ142A/N370A [see e.g. Kriukienė et al. Nature Communications volume 4, Article number: 2190 (2013)].

The cofactor used for labeling of some embodiments is a small molecular weight AdoMet analogue that contain extended unsaturated side chains instead of a methyl group at the sulfonium center.

The extended side chain, which replaces the methyl group in AdoMet, reduces the reaction rate of the transfer by the MTase due to unfavorable steric effects within the transition state. Therefore, in order to accelerate the reaction rate, a double or triple bond may be placed within the transferred chain, next to the reactive carbon atom, which will lead to stabilization of the transition state and hence to a faster reaction rate.

According to a specific embodiment, the cofactor is AdoYnAzide or a derivative thereof.

The cofactor of some embodiments described herein may be attached to a detectable moiety such as a fluorescent moiety as well as any other known detectable moieties as further disclosed hereinabove. Thus, for example, in one embodiment, the cofactor is AdoYnTAMRA, AdoYnAtto532 or AdoYnCF640R.

According to specific embodiments, the label comprises a plurality of labels, for example one for a methylated CpG and the other for an unmethylated CpG. In such embodiments, the different labels are optionally characterized by different absorption, excitation and/or emission wavelengths. In specific embodiments, methylated and de-methylated cytosine residues are labelled with fluorescent labels of green and red emission spectra, respectively.

According to specific embodiments, the method comprises labeling with the CpG site indicative label.

According to specific embodiments, the method comprises labeling CpG sites in the DNA sample following amplification and prior to the contacting on the array.

According to other specific embodiments, the method comprises labeling with a CpG site indicative label following the contacting on the array.

According to specific embodiments, the method further comprises cleaning the surface of the array (e.g., so as to remove DNA molecules not hybridized to the probes) subsequently to contacting on the array, and prior to determining an amount of the label. In some embodiments, cleaning the array is effected by rinsing with a liquid, e.g., an aqueous liquid.

As used herein the term "array" refers to a plurality of probes attached to a microscopic solid surface in an addressable manner.

According to specific embodiments the probes are specific for DNA fragments comprising CpG sites which are distinctive of a cell or tissue type or state. For example, the DNA fragments detected by the probes comprise sequences which are differentially methylated with respect to a second non-identical cell or tissue, thereby allowing identifying the methylation signature of the cell or tissue of interest.

A single microarray can be designed to identify the methylation signature of a single cell or tissue type or state or multiple cells or tissue types or states.

According to specific embodiments, the solid surface is in a form of a slide (e.g., a glass slide, a plastic slide, a silicon slide), for example, a slide such as used for microscopic observation. The slide is optionally configured to be readable by a commercial optical slide reader.

According to specific embodiments, a single array slide may contain multiple printed areas, which may allow testing multiple samples in a single slide.

According to specific embodiments, the array is designed as a grid divided into separated cells (also known as spots, pixels or features) which can be microscopically observed.

In specific embodiments, the grid cells are typically round.

According to specific embodiments, the size of the cells vary between a few nanometers to several hundreds of micrometers.

According to specific embodiments, the grid cells are separated from each other by a space or a spacer of about 50 - 1000 µm.

According to specific embodiments, the grid cells are separated from each other by a space or a spacer of about 100 - 1000 nm.

According to specific embodiments, the grid cells are separated from each other by a space or a spacer of about 500 µm.

According to specific embodiments, the grid cells are separated from each other by a space or a spacer of about 1 - 50 µm.

According to specific embodiments, the array is a traditional solid-phase array wherein each grid cell comprises identical probes.

According to other specific embodiments, the array is designed such that a plurality of different probes are positioned on a single grid cell.

As used herein "plurality of different probes positioned on a single grid cell" refers to non-identical probes directed at a plurality DNA target sequences mixed together in a single grid cell.

It should be noted that as the DNA (e.g. labeled DNA) sample that is contacted with the array was subjected to bisulfite conversion, the probes should not contain cytosine nucleotides which are not in the context of CpG, and thus all such cytosine residues should be replaced with thymidine residues.

Arrays that can be used with specific embodiments of the invention are commercially available from e.g. Illumina, Affymetrix, Agilent.

The sample is contacted with the array under conditions which allow specific hybridization between the probes and the DNA molecules.

As used herein, "hybridization conditions" refer to conditions that promote specific annealing of the probe with its specific DNA target sequence. Such conditions are well-known in the art and include, but not limited to, temperature, buffer, salt, ionic strength, pH, time and the like. Various considerations must be taken into account when selecting the stringency of the hybridization conditions. For example, the more closely the probe reflects the target DNA sequence, the higher the stringency of the assay conditions can be, although the stringency must not be too high so as to prevent hybridization of the probes to the target sequence. Further, the lower the homology of the probes to the target sequence, the lower the stringency of the assay conditions should be, although the stringency must not be too low to allow hybridization to nonspecific DNA sequences. The ability to optimize the reaction conditions is well within the knowledge of one of ordinary skill in the art.

Generally, annealing temperature and timing are determined both by the efficiency with which a probe is expected to anneal to the target and the degree of mismatch that is to be tolerated. The temperature generally ranges from about 37 °C to about 50 °C, and usually from about 40 °C to about 45 °C. Annealing conditions are generally maintained for a period of time ranging from about 1 minute to about 30 minutes, usually from about 1 minute to about 10 minutes.

According to specific embodiments, the hybridization conditions comprise a denaturation step in order to dissociate any double-stranded or hybridized nucleic acid present in the reaction mixture prior to the annealing. The denaturation step generally comprises heating the reaction mixture to an elevated temperature and maintaining the mixture at the elevated temperature for a sufficient period of time. For denaturation, the temperature of the reaction mixture is usually raised to, and maintained at, a temperature ranging from about 85 °C to about 100 °C, usually from about 90 °C to about 98 °C, and more usually from about 93 °C to about 96 °C for a period of time ranging from about 1 to about 30 minutes, usually from about 5 to about 10 minutes.

According to specific embodiments, the hybridization conditions and steps are as disclosed in the Examples section which follows, which serves as an integral part of the specification of the present invention.

Following hybridization, the cells of the grid or array are typically washed to remove unhybridized DNA, and to allow detection of the CpG methylation status characterizing the cells/tissues/organs/fluids represented by the samples.

As detailed herein, in some embodiments the sample DNA is labeled prior to the contacting on the array, and in other embodiments the DNA is labeled subsequent to the contacting on the array, as further detailed hereinabove.

Detection of labeled DNA following hybridization and washing of the cells (or spots) of the grid or array can be performed using any spectrophotometric, chemical and/or enzymatic methods. In specific embodiments, the label is a fluorescent label, and the labeled DNA is detected using a scanner or a fluorescent microscope, and the number of fluorescent spots and intensity, are analyzed by suitable computer image processing software and hardware.

As methylation patterns are unique to each cell type or tissue and can change during pathologic processes (e.g. cancer), the disclosed methods can be used e.g. to identify cell or tissue type or state.

Hence, according to an aspect of the invention there is provided a method of identifying DNA having a methylation pattern distinctive of a cell or tissue type or state, the method comprising determining CpG methylation status in a DNA sample according to the method, wherein said CpG methylation status is indicative of the cell or tissue type or state.

As used herein, the term "distinctive of a cell or tissue type" refers to the differentiation between cells or of multiple cell types-forming a tissue. Examples of cells include, but are not limited to a hepatocyte, a cardiomyocyte, a pancreatic beta cell, a pancreatic exocrine cell, a neuronal cell, a pneumocyte, a podocyte, an endothelial cell, a lymphocyte, an adipocyte, an oligodendrocyte, a skeletal muscle cell and an intestinal epithelial cell.

Also envisaged for the methods disclosed herein are stem cells, progenitor cells, differentiated and undifferentiated cells, pluripotent cells. Cells suitable for analysis with the disclosed methods include, but are not limited to fetal cells, embryonic cells, newborn, child, adolescent, adult and geriatric cells.

The term "tissue" refers to part of an organism consisting of cells designed to perform a function or functions. Examples of tissues include, but are not limited to, liver tissue (comprising e.g. hepatocytes, sinusoidal endothelial cells, phagocytic Kupffer cells and hepatic stellate cells), colon tissue (comprising e.g. simple columnar epithelial cells, enterocytes, Goblet cells, enteroendocrine cells, Paneth cells, microfold cells, cup cells and tuft cells), heart tissue, pancreatic tissue (comprising e.g. exocrine cells, alpha cells, beta cells and delta cells), brain tissue (comprising e.g. neuronal cell and glial cells), lung tissue (comprising e.g. pneumocyts, squamous epithelial cells, goblet cells and club cells), renal tissue (comprising e.g. glomerulus parietal cells, podocytes, proximal tubule brush border cells, loop of Henle thin segment cells, thick ascending limb cells, kidney distal tubule cells collecting duct principal cells, collecting duct intercalated cells and interstitial kidney cells), breast tissue (comprising e.g. epithelial cells, myoepithelial cells and milk-secreting cuboidal cells), retina, skin tissue (comprising e.g. keratinocytes, melanocytes, Merkel cells, and Langerhans cells, mechanoreceptors, endothelial cells, adipocytes and fibroblasts), bone (comprising e.g. osteocytes, osteoblasts and osteoclasts), cartilage, connective tissue, blood tissue (comprising e.g. red blood cells, white blood cells and platelets), bladder tissue (comprising e.g. smooth muscle cells and urothelium cells), prostate tissue (comprising e.g. epithelial cells, smooth muscle cells and fibroblasts), thyroid tissue (comprising e.g. follicular cells and parafollicular cells), ovarian tissue, spleen tissue, muscle tissue, vascular tissue, gonadal tissue, hematopoietic tissue.

In some embodiments, the tissue is selected from the group consisting of liver tissue, colon tissue, heart tissue, pancreatic tissue, brain tissue, lung tissue, renal tissue, breast tissue, bladder tissue, prostate tissue, blood tissue, thyroid tissue, ovarian tissue and spleen tissue.

As used herein, the term "distinctive of a cell or tissue state" refers to the differentiation between a healthy and a pathologic (e.g. cancerous) cell or tissue.

According to specific embodiments, the cell comprises a pathological cell.

In some embodiments, pathological cells are cells from a tissue affected by disease. Non-limiting examples of such diseases include different cancers, autoimmune disorders, neurological disorders or a graft injury that are associated with methylation modifications.

According to specific embodiments, the cell is a cancerous cell.

Cancerous disease, cells or tissue of which can be detected using the methods of some embodiments of the invention include any solid or non-solid cancer and/or cancer metastasis associated with methylation modifications, including, but is not limited to, tumors of the gastrointestinal tract (colon carcinoma, rectal carcinoma, colorectal carcinoma, colorectal cancer, colorectal adenoma, hereditary nonpolyposis type 1, hereditary nonpolyposis type 2, hereditary nonpolyposis type 3, hereditary nonpolyposis type 6; colorectal cancer, hereditary nonpolyposis type 7, small and/or large bowel carcinoma, esophageal carcinoma, tylosis with esophageal cancer, stomach carcinoma, pancreatic carcinoma, pancreatic endocrine tumors), endometrial carcinoma, dermatofibrosarcoma protuberans, gallbladder carcinoma, Biliary tract tumors, prostate cancer, prostate adenocarcinoma, renal cancer (e.g., Wilms' tumor type 2 or type 1), liver cancer (e.g., hepatoblastoma, hepatocellular carcinoma, hepatocellular cancer), bladder cancer, embryonal rhabdomyosarcoma, germ cell tumor, trophoblastic tumor, testicular germ cells tumor, immature teratoma of ovary, uterine, epithelial ovarian, sacrococcygeal tumor, choriocarcinoma, placental site trophoblastic tumor, epithelial adult tumor, ovarian carcinoma, serous ovarian cancer, ovarian sex cord tumors, cervical carcinoma, uterine cervix carcinoma, small-cell and non-small cell lung carcinoma, nasopharyngeal, breast carcinoma (e.g., ductal breast cancer, invasive intraductal breast cancer, sporadic ; breast cancer, susceptibility to breast cancer, type 4 breast cancer, breast cancer-1, breast cancer-3; breast-ovarian cancer), squamous cell carcinoma (e.g., in head and neck), neurogenic tumor, astrocytoma, ganglioblastoma, neuroblastoma, lymphomas (e.g., Hodgkin's disease, non-Hodgkin's lymphoma, B cell, Burkitt, cutaneous T cell, histiocytic, lymphoblastic, T cell, thymic), gliomas, adenocarcinoma, adrenal tumor, hereditary adrenocortical carcinoma, brain malignancy (tumor), various other carcinomas (e.g., bronchogenic large cell, ductal, Ehrlich-Lettre ascites, epidermoid, large cell, Lewis lung, medullary, mucoepidermoid, oat cell, small cell, spindle cell, spinocellular, transitional cell, undifferentiated, carcinosarcoma, choriocarcinoma, cystadenocarcinoma), ependimoblastoma, epithelioma, erythroleukemia (e.g., Friend, lymphoblast), fibrosarcoma, giant cell tumor, glial tumor, glioblastoma (e.g., multiforme, astrocytoma), glioma hepatoma, heterohybridoma, heteromyeloma, histiocytoma, hybridoma (e.g., B cell), hypernephroma, insulinoma, islet tumor, keratoma, leiomyoblastoma, leiomyosarcoma, leukemia (e.g., acute lymphatic, acute lymphoblastic, acute lymphoblastic pre-B cell, acute lymphoblastic T cell leukemia, acute - megakaryoblastic, monocytic, acute myelogenous, acute myeloid, acute myeloid with eosinophilia, B cell, basophilic, chronic myeloid, chronic, B cell, eosinophilic, Friend, granulocytic or myelocytic, hairy cell, lymphocytic, megakaryoblastic, monocytic, monocytic-macrophage, myeloblastic, myeloid, myelomonocytic, plasma cell, pre-B cell, promyelocytic, subacute, T cell, lymphoid neoplasm, predisposition to myeloid malignancy, acute nonlymphocytic leukemia), lymphosarcoma, melanoma, mammary tumor, mastocytoma, medulloblastoma, mesothelioma, metastatic tumor, monocyte tumor, multiple myeloma, myelodysplastic syndrome, myeloma, nephroblastoma, nervous tissue glial tumor, nervous tissue neuronal tumor, neurinoma, neuroblastoma, oligodendroglioma, osteochondroma, osteomyeloma, osteosarcoma (e.g., Ewing's), papilloma, transitional cell, pheochromocytoma, pituitary tumor (invasive), plasmacytoma, retinoblastoma, rhabdomyosarcoma, sarcoma (e.g., Ewing's, histiocytic cell, Jensen, osteogenic, reticulum cell), schwannoma, subcutaneous tumor, teratocarcinoma (e.g., pluripotent), teratoma, testicular tumor, thymoma and trichoepithelioma, gastric cancer, fibrosarcoma, glioblastoma multiforme; multiple glomus tumors, Li-Fraumeni syndrome, liposarcoma, lynch cancer family syndrome II, male germ cell tumor, mast cell leukemia, medullary thyroid, multiple meningioma, endocrine neoplasia myxosarcoma, paraganglioma, familial nonchromaffin, pilomatricoma, papillary, familial and sporadic, rhabdoid predisposition syndrome, familial, rhabdoid tumors, soft tissue sarcoma, and Turcot syndrome with glioblastoma.

According to specific embodiments, the cancer is breast cancer, gastric cancer, liver cancer, esophageal cancer, acute myeloid leukemia, acute lymphocytic leukemia, chronic myeloid leukemia, chronic lymphoblastic leukemia, colorectal cancer and/or lung cancer. The following table shows some different types of cancer and the associated methylation modification target genes:

**Table 2:**

| **Cancer type** | **Gene** | **Promoter methylation** |
|---|---|---|
| Breast | RARB2, MSH2, ESR1B, AKR1B1, COL6A2, GPX7, HIST1H3C, HOXB4, RASGRF2,TM6SF1, ARHGEF7, TMEFF2, RASSF1, BRCA1, STRATIFIN, RASSF1A | Hypermethylation |
| Gastric | RUNX3 | Hypermethylation |
| Liver | CDKN2A | Hypermethylation |
| Esophageal | APC | Hypermethylation |
| Colorectal | SEPT9, hMLH1, CDKN2A/p16, HTLF, ALX4, TMEFF2/HPP1, NGFR, SFRP2, NEUROG1, RUNX3,UBE2Q1 | Hypermethylation |
| Lung | RARB2, RASSF1A, CHFR, STRATI-FIN, SHOX2, RASSF1A APC1 | Hypermethylation |

Non-limiting examples of autoimmune diseases, cells or tissue of which can be detected using the methods of some embodiments of the invention include multiple sclerosis, systemic lupus erythematosus, asthma, Sjogren's syndrome, scleroderma, rheumatoid arthritis, primary biliary cirrhosis, Type I diabetes, psoriasis and ulcerative colitis.

Non-limiting examples of neurodegenerative and psychological disorders, cells or tissue of which can be detected using the methods of some embodiments of the invention include Alzheimer's disease, Huntington's disease, Fragile X syndrome, Autism and psychiatric diseases such as schizophrenia, Rubinstein-Taybi syndrome, bipolar, dementia, alcoholism and addiction, Tatton-Brown, overgrowth syndromes.

According to specific embodiments, the cell has been exposed to an exogenous agent such as chemotherapy, chemical treatment, radiation and DNA damaging agent.

Since the methods of some embodiments of the invention can be used to distinguish between cells, tissue, organs and/or states characteristic of certain pathologies, the methods for detection of DNA CpG methylation status can be used to diagnose pathology as well as treating and monitoring treatment.

Hence, according to an aspect of the invention there is provided a method of diagnosing a pathology in a subject, the method comprising obtaining a biological sample of the subject and identifying DNA having a methylation pattern distinctive of a cell or tissue type or state according to the method disclosed herein, wherein presence and/or level above a predetermined threshold of said DNA having said methylation pattern distinctive of said cell or tissue type or state is indicative of a pathology associated with said cell or tissue in said subject.

According to an additional or an alternative aspect of the invention, there is provided a method of treating a pathology in a subject in need thereof, the method comprising:
(i) diagnosing the pathology in the subject according to the method disclosed herein; and wherein said pathology is indicated
(ii) treating said pathology in said subject.

According to an additional or an alternative aspect of the invention, there is provided a method of monitoring a treatment for a pathology in a subject in need thereof, the method comprising obtaining a biological sample of the subject and identifying DNA having a methylation pattern distinctive of a cell or tissue associated with the pathology according to the method disclosed herein, wherein a decrease above a predetermined threshold of said DNA having said methylation pattern distinctive of said cell or tissue following treatment as compared to same prior to treatment indicates efficacy of treatment of the pathology in said subject.

According to specific embodiments, the method can be used to diagnose, treat, monitor treatment of diseases associated with altered methylation status. Non-limiting Examples of such diseases, including for example cancer, neurological disease, autoimmune disease or graft injury, are further provided hereinabove.

As used herein, the term "diagnosing" refers to determining the presence or absence of a pathology (e.g. a disease, disorder, condition or syndrome), classifying a pathology or a symptom, determining a severity of the pathology, monitoring the pathology's progression, forecasting an outcome of the pathology and/or prospects of recovery and screening of a subject for a specific disease.

In some embodiments, the CpG methylation status distinctive of the cell and/or tissue associated with the pathology is characterized by presence or absence of methylation, which can be of a specific gene(s), of the pathological cells relative to that of healthy cells.

In some embodiments, the CpG methylation status distinctive of the cell and/or tissue associated with the pathology is characterized by a reduction in the extent of methylation, which can be of a specific gene(s), of the pathological cells relative to that of healthy cells.

In some embodiments, the CpG methylation status distinctive of the cell and/or tissue associated with the pathology is characterized by an increase in the extent of methylation, which can be of a specific gene(s), of the pathological cells relative to that of healthy cells.

According to specific embodiments, the decrease or increase is statistically significant.

According to specific embodiments, the predetermined threshold is derived from a control subject, such as a healthy subject or a subject prior to treatment.

According to specific embodiments, the presence and/or level above the predetermined threshold is statistically significant.

According to specific embodiments, the predetermined threshold is a change of at least 1.5 fold or at least 2 fold in the tested sample as compared to a control sample (e.g. obtained from a healthy subject or from a subject prior to treatment).

According to some embodiments of the invention, screening of the subject for a specific disease is followed by substantiation of the screen results using gold standard methods (e.g., biopsy, ultrasound, CT, MRI, TAA expression, cytomorphometry, clinical tissue staining (e.g., Vital iodine stain, Tblue stain).

Methylation patterns are highly stable under physiologic or pathologic conditions. Monitoring of tissue-specific DNA methylation markers in cfDNA has been shown effective for detection of cell death in specific tissues, including pancreatic β-cell death in type 1 diabetes, oligodendrocyte death in relapsing multiple sclerosis, brain cell death in patients after traumatic or ischemic brain damage, and exocrine pancreas cell death in pancreatic cancer or pancreatitis. Thus, according to an aspect of the invention, there is provided a method of detecting death of a cell or tissue of interest in a subject comprising determining whether cell-free DNA (cfDNA) comprised in a fluid sample of the subject is derived from the cell or tissue of interest, wherein said determining is effected by the method disclosed herein, wherein presence and/or level above a predetermined threshold of said DNA having a methylation pattern distinctive of said cell or tissue of interest is indicative of death of the cell or tissue of interest.

According to specific embodiments, when death of the cell or tissue is associated with a pathology, the method further comprises diagnosing the pathology.

cfDNA derives, for the most part, from dead cells, and blood levels of cfDNA are known to increase in many conditions, for example, traumatic brain injury, cardiovascular disease, sepsis and intensive exercise. Thus, in specific embodiments, the distinctive methylation status is discerned in the cfDNA of a sample or samples from the subject.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the terms "treating" and "treatment" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### DEVELOPMENT OF A NOVEL METHOD FOR DETERMINING CpG METHYLATION STATUS OF DNA

### MATERIALS AND METHODS

***Extraction of circulating cell-free DNA*** - Circulating cell-free DNA (cfDNA) was extracted using the "Plasma/Serum Cell-Free Circulating DNA Purification Midi Kit (NORGEN, Cat. 55600), according to the manufacturer's instructions. This kit employs a two-column method for the isolation of high-quality, high-purity, and inhibitor-free cfDNA from fresh or frozen plasma/serum samples. Briefly, five mL of serum were used as an input volume on the first column to extract cfDNA, which was concentrated on the second mini column into a final elution volume of 30 µl. The cfDNA obtained was stored at 4 °C.

***NEBNext Ultra II End Repair* / *dA-Tailing module -*** cfDNA was end-repaired using NEBNext Ultra II End-Repair/dA-tailing Module (NEB, Cat. E7546), according to the manufacturer's instructions. Briefly, the cfDNA was converted by the NEBNext End Repair Module to blunt ended DNA having 5'-phosphates and 3'-hydroxyl. DNA repaired by the NEBNext End Repair Module was subsequently converted to DNA having 3' dA-tails with the NEBNext dA-tailing Module. A mixture of 5 µL 10 mM Tris-EDTA (TE), 20 µL cfDNA, 1.5 µL NEBNext Ultra II End Prep Enzyme Mix and 3.5 µL NEBNext Ultra II End Prep Reaction Buffer was placed in a thermocycler for 30 minutes at 20 °C followed by 30 minutes at 65 °C.

***Adaptors and primers design -*** The adaptors were custom designed to allow the uniform amplification of the cfDNA. Importantly, the adaptors were designed such that following the bisulfite treatment they will remain unchanged, hence, all cytosines in the adaptors' sequence were methylated. Moreover, in order to avoid the labeling of the adaptors with a fluorescent signal, the adaptors were designed without CpGs sequences. These sequences of the two adaptors, and their respective primers is shown in Table 1 hereinbelow.

**Table 1: List of adaptors and their respective primers**

| | | |
|---|---|---|
| Adaptor 1 | 5-GTCTAGGGAACATAGGATCAGGACT | SEQ ID NO: 1 |
| Primer 1 | GTCTAGGGAACATAGGATCAG | SEQ ID NO: 2 |
| Adaptor 2 | 5-*P-GTCCTGATCCTATTAATGACTACAACTTG | SEQ ID NO: 3 |
| Primer 2 | CAAGTTGTAGTCATTAATAGG | SEQ ID NO: 4 |

| | | |
|---|---|---|
| * P at the beginning of the sequence represents a phosphate group | | |

***Ligation of Adaptors* -** Ligation of the costume-designed Adaptors was performed with the NEBNext Ultra II Ligation Module (NEB, Cat. E7595). 30 µL of the End-prepared DNA was mixed with 15 µL NEBNext Ultra II Ligation Master Mix, 0.5 µL NEBNext Ligation Enhancer, and 0.5 µL of each of the two adaptors (10 µM, final concentration of 0.25 µM). The mixture was incubated for 15 minutes at 20°C. Following ligation, the DNA was purified from excess of adaptors using the Select-a-Size DNA Clean & Concentrator MagBead Kit (ZYMO, Cat. D4084), according to the manufacturer's instructions. In order to retain DNA fragments larger than 150 bp, 50 µL of sample was mixed with 60 µL of Select-a-Size MagBead Buffer. DNA was washed twice and eluted in 20 µL of DNA Elution Buffer.

***Sodium Bisulfite treatment -*** In order to convert all unmethylated cytosine nucleotides into uracil nucleotides, the ligated cfDNA was treated with sodium bisulfite, using the EZ DNA Methylation-Gold Kit (ZYMO, Cat. D5006), according to the manufacturer's instructions, with minor changes. 20 µL of ligated and purified cfDNA was mixed with 130 µL of CT conversion buffer, and was placed in a thermocycler for 10 minutes at 98 °C followed by 105 minutes at 64 °C. DNA was then purified as described in the manufacturer's instructions, and was eluted in 20 µL.

***PCR amplification** -* The purified bisulfite treated cfDNA was amplified using the costume made primers (Table 1 hereinabove). 20 µL of bisulfite treated cfDNA was mixed with 25 µL of MyTaq Red Mix (Bioline, Cat. BIO-25043) and 3 µL of each of the two primers (10 µM, final concentration of 1.8 µM). The mixture was placed in a thermocycler for the following program:

| Step | Temperature | Time | Cycles |
|---|---|---|---|
| Initial denaturation | 95°C | 1 minute | 1 |
| Denaturation | 95°C | 20 seconds | 32 |
| Annealing | 55°C | 20 seconds | |
| Extension | 72°C | 20 seconds | |
| Final extension | 72°C | 6 minutes | 1 |

PCR products were purified first by QIAquick PCR Purification Kit (QIAGEN, Cat. 28106), according to the manufacturer's instructions, then by the Select-a-Size DNA Clean & Concentrator MagBead Kit (ZYMO, Cat. D4084), as described above under "ligation of adaptors". Purified DNA was eluted in 25-50 µL and stored at 4 °C.

***Unmethylated CpGs labeling -*** The amplified DNA was labeled in a two-step chemoenzymatic reaction. In the first step, the M.mpeI double-mutant enzyme was used [Giesbertz, A., Kiss, A., & Weinhold, E. (2019). Triple helix-targeted DNA methylation with DNA methyltransferase-oligodeoxynucleotide conjugates (No. RWTH-2019-00264). Fachgruppe Chemie.]. The synthetic cofactor used is named AdoYnAzide, and it contains an active azide moiety (N₃), which is a chemically reactive group. In the second step, a fluorophore was covalently conjugated to the AdoYnAzide via a high-efficient click chemistry reaction. First, 1 µg of amplified DNA was mixed with 0.5 µL of AdoYnAzide (80 µM final concentration), 2 µL of 50 % glycerol (5 % final concentration), 0.2 µL BSA (0.1 mg / mL final concentration), 2 µL of 2-mercaptoethanol (10 mM final concentration), 2 µL of M.mpeI buffer X10 (50 mM Tris-HCl, 100 mM NaCl, 5 % glycerol) and 2 µL of M.mpeI double mutant (5 µM final concentration). The mixture was incubated for 4 hours at 37 °C. Following, 1 µL of protein K 20 mg/mL (Sigma) was added, and the reaction was incubated for an additional hour at 37 °C. Finally, 0.5 µL of Dibenzocyclooctyl (DBCO)-PEG4-5/6-TAMRA (Jena Bioscience, Jena, Germany) was added to a final concentration of 150 µM, and the reaction was incubated over-night at 37 °C. The labeled DNA samples were purified from excess fluorophores using Oligo Clean & Concentrator (Zymo research, Cat. D4060), according to manufacturer's recommendations, with two washing steps for optimal results.

***Slide microarray design*** - The custom-made microarray used was manufactured by Phalanx Biotech. Specific short sequences where bioinformatically chosen by their unique methylation pattern in specific CpG islands. This methylation pattern is unique to each cell type or tissue, and can change during pathologic processes (e.g. cancer). Figures 1A-B show an example of such an array, which contains 28 different locations, each with four spots (pixels) of a single type of sequence, and additional two locations, each with four spots, each contains a combination of 3-6 sequences, each type of location is specific to a single organ.

***Commercial microarray** -* Human Genome CGH Microarray (Agilent, Cat No. G4827A), containing 60K oligo sequences, including known genes, promoters, miRNAs, PAR and telomeric regions.

***Hybridization to the slide microarray -*** In order to hybridize double-stranded DNA (dsDNA) to the microarray, a unique procedure of heating-cooling of the dsDNA as well as of the microarray was used. Most known assays for microarray hybridization use either RNA or ssDNA, the procedure used enables using dsDNA, and thus avoiding additional steps to turn dsDNA into ssDNA. First, the microarray slide was incubated in a pre-hybridization buffer (20X SSC, 20 % SDS, 5 % BSA) for 20 minutes in 65 °C. Following, the dsDNA was placed in hybridization solution (20X SSC, 20 % SDS) and incubated for 5 minutes in 95°C. Finally, the microarray slide was heated in a slide compartment to 42 °C, followed by immediate addition of the 95 °C dsDNA into the specific array location. The slide was then incubated for 4 hours in the slide compartment. Following incubation, the slide was washed with two consecutive wash buffers, "wash buffer A" (1.25 mL, 50 µL 20X SSC, 20 % SDS, 47.5 mL purified water) and "wash buffer B" (135 µL 20X SSC, 90 mL purified water). Following, the slide was dried under a nitrogen stream, and immediately imaged.

***Slide imaging** -* Slides were imaged using InnoScan1100 slide scanner (Innopsys). A 532 nm green laser was used to image the DBCO-TAMRA fluorophore. Scanning parameters were optimized to fit the entire range of fluorescence intensities on the scanned slide and to avoid technical artifacts such as saturation and photomultiplier nonlinearity.

### RESULTS

cfDNA was extracted from the blood of a healthy individual, and was then ligated with costume made adaptors, to allow for the uniform and non-biased amplification of the cfDNA. Following, the DNA went through bisulfite treatment, to convert all unmethylated cytosine nucleotides into uracil nucleotides. Following, all the bisulfite treated DNA was amplified. During the amplification process, all uracil nucleotides (i.e. cytosine nucleotides that were unmethylated in the cfDNA prior to the bisulfite conversion) became thymidine nucleotides. Hence, following amplification only CpGs that were originally methylated in the cfDNA sample remained as CpGs without a methyl attached to them. Subsequently, fluorescent labeling of unmethylated CpGs was performed by a two-step chemoenzymatic reaction. A CpG methyltransferase was used *in-vitro* together with a synthetic cofactor to attach a fluorophore to the unmethylated CpG sites. The methyltransferase used was a M.MpeI double mutant enzyme that catalyzes the addition of a synthetic cofactor within the CpG sequence, instead of the methyl group. The sample was then hybridized to a custom designed microarray, which contains the capture probes of interest. The spots which light up in the array represent the sequences which were labeled and hybridized to the slide (Figure 2).

As the methylation pattern is unique to each cell type or tissue and can change during pathologic processes (e.g. cancer), such a method can further be used to e.g. identify cell or tissue type or state. To this end, cfDNA was extracted from the blood of four healthy individual, four colon cancer patients, and three blood cancer patients and subjected to the same process described hereinabove. Namely, samples were ligated with custom made adaptors, followed by bisulfite treatment and amplification. Subsequently, fluorescent labeling of unmethylated CpGs was performed by the described two-step chemoenzymatic reaction and the samples were then hybridized to a commercial microarray (Human Genome CGH Microarray, Agilent). Data from different arrays was normalized and loci with over two-fold differential methylation between healthy and cancer samples were indicated as potential biomarkers. For the colon cancer samples, 1684 such loci (out of ^{~}60,000 loci, Figure 3A) were identified. 1382 of these loci coincide with genes, and over 150 showed very significant differences of over ten-fold between samples obtained from cancer and healthy patients (Figures 3B-C). For the blood cancer samples, 1263 such loci (out of ^{~}60,000 loci, Figure 4) were identified. 757 of these loci coincide with genes.

### EXAMPLE 2

### DEVELOPMENT OF A NOVEL METHOD FOR DETERMINING CpG METHYLATION STATUS OF DNA - A COMPARISON BETWEEN ADAPTOR'S BASED AMPLIFICATION AND ISOTHERMAL NUCLEIC ACID AMPLIFICATION MATERIALS AND METHODS

***Adaptors-based amplification (protocol 1):** Extraction of circulating cell-free DNA, NEBNext Ultra II End Repair* / *dA-Tailing module, Adaptors and primers design, Ligation of Adaptors, Sodium Bisulfite treatment, PCR amplification -* as described in Examples 1 hereinabove.

***Isotheraml nucleic acid amplification (Protocol 2):*** Isothermal nucleic acid amplification was effected according to Infinium HD Methylation Assay (Illumina kit, Cat No. WG-317- 1002) manufacturer's instructions. Briefly:
*Sodium Bisulfite treatment -* In order to convert all unmethylated cytosine nucleotides into uracil nucleotides, 500 ng of genomic DNA (blood DNA, and U2OS cell line DNA) was treated with sodium bisulfite, using the EZ DNA Methylation-Gold Kit (ZYMO, Cat. D5006), according to manufacturer's protocol. 20 µL of genomic DNA was mixed with 130 µL of CT conversion buffer, and was placed in a thermocycler for 30 seconds at 95 °C followed by 60 minutes at 50 °C, for 16 consecutive cycles. DNA was then purified as described in the ZYMO manufacturer's instructions, and was eluted in 20 µL.
*DNA amplification* - Bisulfite-treated DNA was amplified according to the Illumina kit protocol. Four µL of bisulfite treated DNA sample was mixed with four µL of 0.1M NaOH, vortexed and incubated at room temperature for 10 minutes. 68 µL of RPM and 75 µL of MSM were added to the sample, which was vortexed and incubated for 24 hours at 37°C.
*Fragment DNA-* Amplified DNA was fragmented according to the Illumina kit protocol. 50 µL of FMS were added to the amplified DNA sample, which was vortexed and incubated for one hour at 37 °C.

***Comparison of amplification protocols -*** To compare the amplification efficiency of the two protocols, the amplification products (50 ng) of both protocols were used as templates in amplifying a known human DNA segment. To this end, DNA was mixed with 25 µL of MyTaq Red Mix (Bioline, Cat. BIO-25043) and 3 µL of each of the two primers (10 µM, final concentration of 1.8 µM)@
Forward Primer sequence: 5'- GTTTGGTAATTTATTTAGAGAAGTAAAATGAT (SEQ ID NO: 9)
Reverse Primer Sequence: 5'- TACAAATCCCACAAATAAAAAAAATACT (SEQ ID NO: 10) The mixture was placed in a thermocycler for the following program:

| Step | Temperature | Time | Cycles |
|---|---|---|---|
| Initial denaturation | 95°C | 1 minute | 1 |
| Denaturation | 95°C | 20 seconds | 32 |
| Annealing | 55°C | 20 seconds | |
| Extension | 72°C | 20 seconds | |
| Final extension | 72°C | 6 minutes | 1 |

Following, the amplification products were ran in a gel electrophoresis, to estimate the quantity of the specific product. The expected length of the DNA segment amplified is 100 bp. A 2% agarose gel was prepared by dissolving 1.2 gr of agarose (Cat. 889153, TAU storage) in 60 mL of TBE. Six µL of SYBR safe (Cat. S33102, Invitrogen) were added to the mixture, which was poured into a gel electrophoresis device. The gel was left to stabilize for ~30 minutes, after which the relevant DNA samples were inserted, as well as a 100 bp ladder (Cat. N3231L, NEB). Gel was ran in 100V for 45 minutes, to receive optimal separation.

### RESULTS

To compare the amplification efficiency of two protocols effected following sodium bisulfite conversion, one that is based on adaptors' ligation (protocol 1) and the other based on isothermal nucleic acid amplification (by phi29, protocol 2, according to the Illumina kit protocol), the amplification products of both protocols were used as templates in amplifying a known human DNA segment. Following, the amplification products were ran in a gel electrophoresis, to estimate the quantity of the specific product. Measuring the intensity of a band at the expected product size of 100 bp, it was found that protocol 1 amplified template yielded a six-times stronger band, than that of protocol 2 (Figure 5); indicating that the amplification method effected according to protocol 1 and based on adaptors ligation, is more efficient and specific than the amplification method effected according to protocol 2 that is based on isotheraml nucleic acid amplification.

Although the invention has been described in conjunction with specific embodiments thereof, it is evident that many alternatives, modifications and variations will be apparent to those skilled in the art.

## Claims

1. A method of determining CpG methylation status in a DNA sample, the method comprising:
(a) subjecting the DNA sample to bisulfite conversion;
(b) amplifying said DNA sample following said (a) by a PCR using adaptors ligation, to obtain an amplified DNA sample;
(c) labeling CpG sites in said amplified DNA sample with a label using a CpG methylation-sensitive methyltransferase (MTase), to obtain a labeled DNA sample;
(d) contacting said amplified DNA sample on an array comprising a plurality of probes for said DNA under conditions which allow specific hybridization between said plurality of probes and said DNA; and
(e) detecting said hybridization, wherein an amount of said label is indicative of the CpG methylation status in said DNA sample.

2. The method of claim 1, wherein said DNA sample comprises DNA fragments, and optionally wherein said DNA fragments are about 100-300 nucleotides long.

3. The method of claim 1, comprising fragmenting said DNA so as to obtain DNA fragments prior to said contacting.

4. The method of any one of claims 1-3, wherein a concentration of said DNA prior to amplification in said sample is ≥ 0.01 pg per ml.

5. The method of any one of claims 1-4, wherein said DNA is cell-free DNA (cfDNA).

6. The method of any one of claims 1-5, wherein said adaptors comprise a methylated cytosine nucleotide.

7. The method of any one of claims 1-6, wherein said adaptors are devoid of a CpG site.

8. The method of any one of claims 1-7, wherein said adaptors are devoid of an unmethylated cytosine nucleotide.

9. The method of any one of claims 1-8, wherein said labeling is effected prior to said contacting.

10. The method of any one of claims 1-8, wherein said labeling is effected following said contacting.

11. The method of any one of claims 1-10, wherein ligation of said adaptors to said DNA sample is effected prior to said subjecting.

12. A method of identifying DNA having a methylation pattern distinctive of a cell or tissue type or state, the method comprising determining CpG methylation status in a DNA sample according to the method of anyone of claims 1-11, wherein said CpG methylation status is indicative of the cell or tissue type or state.

13. A method of diagnosing a pathology in a subject, the method comprising identifying in a biological sample of the subject DNA having a methylation pattern distinctive of a cell or tissue type or state according to the method of claim 12, wherein presence and/or level above a predetermined threshold of said DNA having said methylation pattern distinctive of said cell or tissue type or state is indicative of a pathology associated with said cell or tissue in said subject.

14. A method of monitoring a treatment for a pathology in a subject in need thereof, the method comprising identifying in a biological sample of the subject DNA having a methylation pattern distinctive of a cell or tissue associated with the pathology according to the method of claim 12, wherein a decrease above a predetermined threshold of said DNA having said methylation pattern distinctive of said cell or tissue following treatment as compared to same prior to treatment indicates efficacy of treatment of the pathology in said subject.

15. A method of detecting death of a cell or tissue of interest in a subject, the method comprising determining whether cell-free DNA (cfDNA) comprised in a fluid sample of the subject is derived from the cell or tissue of interest, wherein said determining is effected by the method of claim 12, wherein presence and/or level above a predetermined threshold of said DNA having a methylation pattern distinctive of said cell or tissue of interest is indicative of death of the cell or tissue of interest.

## Patentansprüche

1. Verfahren zum Bestimmen des CpG-Methylierungsstatus in einer DNA-Probe, wobei das Verfahren Folgendes umfasst:
(a) Unterziehen der DNA-Probe einer Bisulfit-Umwandlung;
(b) Amplifizieren der DNA-Probe nach (a) durch eine PCR unter Verwendung von Adapterligation, um eine amplifizierte DNA-Probe zu erhalten;
(c) Markieren von CpG-Stellen in der amplifizierten DNA-Probe mit einer Markierung unter Verwendung einer CpG-methylierungssensitiven Methyltransferase (MTase), um eine markierte DNA-Probe zu erzielen;
(d) Kontaktieren der amplifizierten DNA-Probe auf einem Array, das eine Vielzahl von Sonden für die DNA umfasst, unter Bedingungen, die spezifische Hybridisierung zwischen der Vielzahl von Sonden und der DNA ermöglichen; und
(e) Nachweisen der Hybridisierung, wobei eine Menge der Markierung den CpG-Methylierungsstatus in der DNA-Probe anzeigt.

2. Verfahren nach Anspruch 1, wobei die DNA-Probe DNA-Fragmente umfasst, und optional, wobei die DNA-Fragmente etwa 100-300 Nucleotide lang sind.

3. Verfahren nach Anspruch 1, umfassend das Fragmentieren der DNA, um vor dem Kontaktieren DNA-Fragmente zu erhalten.

4. Verfahren nach einem der Ansprüche 1-3, wobei eine Konzentration der DNA vor Amplifikation in der Probe ≥ 0,01 pg pro ml beträgt.

5. Verfahren nach einem der Ansprüche 1-4, wobei die DNA zellfreie DNA (cfDNA) ist.

6. Verfahren nach einem der Ansprüche 1-5, wobei die Adapter ein methyliertes Cytosin-Nukleotid umfassen.

7. Verfahren nach einem der Ansprüche 1-6, wobei die Adapter frei von CpG-Stellen sind.

8. Verfahren nach einem der Ansprüche 1-7, wobei die Adapter frei von einem unmethylierten Cytosin-Nukleotid sind.

9. Verfahren nach einem der Ansprüche 1-8, wobei das Markieren vor dem Kontaktieren vorgenommen wird.

10. Verfahren nach einem der Ansprüche 1-8, wobei das Markieren nach dem Kontaktieren vorgenommen wird.

11. Verfahren nach einem der Ansprüche 1-10, wobei die Ligation der Adapter an die DNA-Probe vor dem Unterziehen vorgenommen wird.

12. Verfahren zum Identifizieren von DNA, die ein Methylierungsmuster aufweist, das für einen Zell- oder Gewebetyp oder -zustand unterscheidend ist, wobei das Verfahren das Bestimmen des CpG-Methylierungsstatus in einer DNA-Probe gemäß dem Verfahren nach einem der Ansprüche 1-11 umfasst, wobei der CpG-Methylierungsstatus anzeigend ist für den Zell- oder Gewebetyp oder -zustand.

13. Verfahren zum Diagnostizieren einer Pathologie in einem Subjekt, wobei das Verfahren das Identifizieren in einer biologischen Probe des Subjekts von DNA umfasst, die ein Methylierungsmuster aufweist, das für einen Zell- oder Gewebetyp oder -zustand unterscheidend ist, gemäß dem Verfahren nach Anspruch 12, wobei Anwesenheit und/oder ein Spiegel oberhalb eines vorbestimmten Schwellenwerts der DNA, die das Methylierungsmuster aufweist, das für den Zell- oder Gewebetyp oder -zustand unterscheidend ist, eine Pathologie anzeigt, die der Zelle oder dem Gewebe in dem Subjekt zugeordnet ist.

14. Verfahren zur Überwachung einer Behandlung für eine Pathologie in einem Subjekt, das diese benötigt, wobei das Verfahren das Identifizieren in einer biologischen Probe des Subjekts von DNA umfasst, die ein Methylierungsmuster aufweist, das für eine Zelle oder ein Gewebe unterscheidend ist, das der Pathologie zugeordnet ist, gemäß dem Verfahren nach Anspruch 12, wobei eine Verringerung über einen vorbestimmten Schwellenwert der DNA, die das Methylierungsmuster aufweist, das für die Zelle oder das Gewebe unterscheidend ist, nach der Behandlung im Vergleich damit vor der Behandlung die Wirksamkeit der Behandlung der Pathologie in dem Subjekt anzeigt.

15. Verfahren zum Nachweisen des Todes einer Zelle oder eines Gewebes von Interesse in einem Subjekt, wobei das Verfahren das Bestimmen umfasst, ob zellfreie DNA (cfDNA), die in einer Fluidprobe des Subjekts enthalten ist, von der Zelle oder dem Gewebe von Interesse abgeleitet ist, wobei das Bestimmen durch das Verfahren nach Anspruch 12 vorgenommen wird, wobei die Anwesenheit und/oder ein Spiegel oberhalb eines vorbestimmten Schwellenwerts der DNA, die das Methylierungsmuster aufweist, das für die Zelle oder das Gewebe von Interesse unterscheidend ist, den Tod der Zelle oder des Gewebes von Interesse anzeigt.

## Revendications

1. Procédé de détermination du statut de méthylation des CpG dans un échantillon d'ADN, le procédé comprenant :
(a) la soumission de l'échantillon d'ADN à une conversion au bisulfite ;
(b) l'amplification dudit échantillon d'ADN suite à ladite (a) par une PCR en utilisant une ligature d'adaptateurs, pour obtenir un échantillon d'ADN amplifié ;
(c) le marquage des sites de CpG dans ledit échantillon d'ADN amplifié avec un marqueur en utilisant une méthyltransférase (MTase) sensible à la méthylation de CpG, pour obtenir un échantillon d'ADN marqué ;
(d) la mise en contact dudit échantillon d'ADN amplifié sur un réseau comprenant une pluralité de sondes pour ledit ADN dans des conditions qui permettent une hybridation spécifique entre ladite pluralité de sondes et ledit ADN ; et
(e) la détection de ladite hybridation, dans lequel une quantité dudit marqueur est indicative du statut de méthylation des CpG dans ledit échantillon d'ADN.

2. Procédé selon la revendication 1, dans lequel ledit échantillon d'ADN comprend des fragments d'ADN, et facultativement dans lequel lesdits fragments d'ADN font environ 100 à 300 nucléotides de long.

3. Procédé selon la revendication 1, comprenant la fragmentation dudit ADN de manière à obtenir des fragments d'ADN avant ladite mise en contact.

4. Procédé selon l'une quelconque des revendications 1 à 3, dans lequel une concentration dudit ADN avant amplification dans ledit échantillon est ≥ 0,01 pg par ml.

5. Procédé selon l'une quelconque des revendications 1 à 4, dans lequel ledit ADN est de l'ADN acellulaire (ADNcf).

6. Procédé selon l'une quelconque des revendications 1 à 5, dans lequel lesdits adaptateurs comprennent un nucléotide de cytosine méthylée.

7. Procédé selon l'une quelconque des revendications 1 à 6, dans lequel lesdits adaptateurs sont dépourvus d'un site de CpG.

8. Procédé selon l'une quelconque des revendications 1 à 7, dans lequel lesdits adaptateurs sont dépourvus d'un nucléotide de cytosine non méthylée.

9. Procédé selon l'une quelconque des revendications 1 à 8, dans lequel ledit marquage est effectué avant ladite mise en contact.

10. Procédé selon l'une quelconque des revendications 1 à 8, dans lequel ledit marquage est effectué suite à ladite mise en contact.

11. Procédé selon l'une quelconque des revendications 1 à 10, dans lequel la ligature desdits adaptateurs audit échantillon d'ADN est effectuée avant ladite soumission.

12. Procédé d'identification d'ADN présentant un modèle de méthylation distinctif d'un type ou d'un état de cellule ou de tissu, le procédé comprenant la détermination du statut de méthylation des CpG dans un échantillon d'ADN selon le procédé selon l'une quelconque des revendications 1 à 11, dans lequel ledit statut de méthylation des CpG est indicatif du type ou de l'état de la cellule ou du tissu.

13. Procédé de diagnostic d'une pathologie chez un sujet, le procédé comprenant l'identification dans un échantillon biologique du sujet d'ADN présentant un modèle de méthylation distinctif d'un type ou d'un état de cellule ou de tissu selon le procédé selon la revendication 12, dans lequel la présence et/ou le niveau au-dessus d'un seuil prédéterminé dudit ADN présentant ledit modèle de méthylation distinctif dudit type ou dudit état de cellule ou de tissu sont indicatifs d'une pathologie associée à ladite cellule ou audit tissu chez ledit sujet.

14. Procédé de surveillance d'un traitement pour une pathologie chez un sujet en ayant besoin, le procédé comprenant l'identification dans un échantillon biologique du sujet d'ADN présentant un modèle de méthylation distinctif d'une cellule ou d'un tissu associé à la pathologie selon le procédé selon la revendication 12, dans lequel une diminution au-dessus d'un seuil prédéterminé dudit ADN présentant ledit modèle de méthylation distinctif de ladite cellule ou dudit tissu après traitement par rapport à celui-ci avant traitement indique l'efficacité du traitement de la pathologie chez ledit sujet.

15. Procédé de détection de la mort d'une cellule ou d'un tissu d'intérêt chez un sujet, le procédé comprenant le fait de déterminer si l'ADN acellulaire (ADNcf) compris dans un échantillon fluide du sujet est dérivé de la cellule ou du tissu d'intérêt, dans lequel ladite détermination est effectuée par le procédé selon la revendication 12, dans lequel la présence et/ou le niveau au-dessus d'un seuil prédéterminé dudit ADN présentant un modèle de méthylation distinctif de ladite cellule ou dudit tissu d'intérêt sont indicatifs de la mort de la cellule ou du tissu d'intérêt.
